Europäisches Patentamt

European Patent Office

Office européen des brevets

(11) Veröffentlichungsnummer: **0 436 860 A1**

# EUROPÄISCHE PATENTANMELDUNG

(21) Anmeldenummer: 90123914.5

(22) Anmeldetag: **12.12.90**

(51) Int. Cl.⁵: **C07C 57/58**, C07C 51/00

(30) Priorität: **23.12.89 DE 3942790**

(43) Veröffentlichungstag der Anmeldung:
**17.07.91 Patentblatt 91/29**

(84) Benannte Vertragsstaaten:
**AT BE CH DE ES FR GB IT LI NL SE**

(71) Anmelder: **HOECHST AKTIENGESELLSCHAFT
Postfach 80 03 20
W-6230 Frankfurt am Main 80(DE)**

(72) Erfinder: **Weber, Jürgen, Dr. Dipl.-Chem.
Bunsenstrasse 17
W-4200 Oberhausen 11(DE)**
Erfinder: **Lappe, Peter, Dr. Dipl.-Chem.
Eickenhof 34
W-4220 Dinslaken(DE)**
Erfinder: **Springer, Helmut, Dipl.-Chem.
Borbeckerstrasse 19
W-4200 Oberhausen 11(DE)**

(54) Verfahren zur Herstellung von 2-(4-Chlorphenyl)-3-methyl-buttersäure.

(57) Die Erfindung betrifft ein neues Verfahren zur Herstellung von 2-(4-Chlorphenyl)-3-methylbuttersäure aus 4-Chlorbenzaldehyd über die Zwischenstufen 3-(4-Chlorphenyl)-2-methylpropenal, 3-(4-Chlorphenyl)-2-methylpropanol, 1-(4-Chlorphenyl)-2-methylpropen-1 und 2-(4-Chlorphenyl)-2-methylbutyraldehyd.

# VERFAHREN ZUR HERSTELLUNG VON 2-(4-CHLORPHENYL)-3-METHYLBUTTERSÄURE

Die Erfindung betrifft ein neues Verfahren zur Herstellung von 2-(4-Chlorphenyl)-3-methylbuttersäure.

Die genannte Verbindung zählt zur Klasse der substituierten Phenylessigsäurederivate, die wertvolle Zwischenprodukte für Insektizide, Arzneimittel und Agrarchemikalien darstellen. Entsprechend ihrer Verwendung wird auf hohe Reinheit dieser Verbindungen Wert gelegt, weil Verunreinigungen die Qualität und die Wirksamkeit der Endprodukte in starkem Maße beeinträchtigen.

Zur Herstellung substituierter Phenylessigsäurederivate und insbesondere der 2-(4-Chlorphenyl)-3-methylbuttersäure sind verschiedene Verfahren bekannt.

So setzt man z.B. nach GB 15 28 043 4-Halogenbenzylhalogenide $4-X-C_6H_4-CH_2X$ (X ist gleich oder verschieden und steht für Cl, Br oder J) etwa mit der äquimolaren Menge eines Alkalicyanids und einer quaternären Ammoniumverbindung um. Es entsteht ein Benzylnitrilderivat, das mit einem molaren Überschuß eines sekundären Alkylchlorids in Gegenwart von Alkalihydroxid zur Reaktion gebracht wird und anschließend zur Carbonsäure verseift werden kann.

Nach C.A. 105 (1986), 78660w, methyleniert man 4-Chlorphenylisopropylketon mit einem Sulfoniumsalz, isomerisiert das resultierende Epoxid mit einer Lewis- oder Brønstedsäure und oxidiert den erhaltenen Aldehyd zu 2-(4-Chlorphenyl)-3-methylbuttersäure.

Schließlich wird in der veröffentlichten japanischen Patentanmeldung 79 84.543 die Herstellung von 2-(4-Chlorphenyl)-3-methylbuttersäure aus 4-Chlorphenyl-2-methylpropylketon beschrieben. Durch Umsetzung mit einem sekundären Amin und einem Dehydratisierungsmittel wird das Keton in ein Enamin überführt. Seine Reaktion mit dem Azid eines Phosphorsäureesters ergibt ein Amidin, dessen Hydrolyse zu 2-(4-Chlorphenyl)-3-methylbuttersäure führt.

Die bekannten Verfahren erfüllen noch nicht alle Ansprüche, die an ein modernes, kommerziell genutztes chemisches Verfahren gestellt werden. Neben der bereits erwähnten hohen Reinheit des Reaktionsproduktes wird insbesondere erwartet, daß die Ausgangsstoffe leicht zugänglich sind, keine, die Umwelt belastenden Nebenprodukte entstehen und zumindest gute Ausbeuten erzielt werden.

Es bestand daher die Aufgabe ein Verfahren zu entwickeln, das diesen Forderungen genügt, wirtschaftlich ist und technisch problemlos durchgeführt werden kann.

Erfindungsgemäß wird diese Aufgabe gelöst durch ein Verfahren zur Herstellung von 2-(4-Chlorphenyl)-3-methylbuttersäure. Es ist dadurch gekennzeichnet, daß man 4-Chlorbenzaldehyd mit Propionaldehyd zu 3-(4-Chlorphenyl)-2-methylpropenal umsetzt, das Propenalderivat unter Bildung von 3-(4-Chlorphenyl)-2-methylpropanol hydriert, den Alkohol dehydratisiert, wobei 1-(4-Chlorphenyl)-2-methylpropen-1 entsteht, dieses substituierte Propylen hydroformyliert und den dabei entstehenden 2-(4-Chlorphenyl)-3-methylbutyraldehyd zur 2-(4-Chlorphenyl)-3-methylbuttersäure oxidiert.

Die neue Arbeitsweise zeichnet sich dadurch aus, daß sie preiswerte, in technischen Mengen verfügbare Ausgangsmaterialien einsetzt, technisch leicht zu realisierende Reak tionsschritte enthält und das gewünschte Produkt rein und in guten Ausbeuten erhalten wird. Umweltbelastende Abfallstoffe entstehen nicht.

Die unter Wasserabspaltung verlaufende Aldolkondensation des 4-Chlorbenzaldehyds mit dem Propionaldehyd erfolgt unter dem katalytischen Einfluß von Alkalihydroxiden, Alkalicarbonaten oder Aminen, vorzugsweise von Alkalihydroxiden wie NaOH. Die beiden Aldehyde werden im Molverhältnis 1 : 1 bis 1 : 2, vorzugsweise 1 : 1,1 bis 1 : 1,3 eingesetzt. Die Katalysatormenge beträgt 0,1 bis 0,5 mol je mol 4-Chlorbenzaldehyd. Alkalihydroxid und Alkalicarbonat werden üblicherweise als wäßrige Lösungen angewandt. Es empfiehlt sich, Lösungen einzusetzen, die die Alkaliverbindungen in Mengen von 10 bis 100 g, vorzugsweise 25 bis 75 g je Liter Lösung enthalten. Üblicherweise vollzieht sich die Umsetzung bei Temperaturen von 80 bis 120, vorzugsweise 95 bis 105°C. Die Ausbeute liegt, bezogen auf 4-Chlorbenzaldehyd, bei annähernd 90 % der Theorie. Die Ausgangsstoffe können in der handelsüblichen Reinheit verwendet werden.

Zur Isolierung des 3-(4-Chlorphenyl)-2-methylpropenals trennt man die organische von der wäßrigen Phase ab. Für die Weiterverarbeitung reicht es aus, das Produkt einmal mit Wasser zu waschen.

Im folgenden Reaktionsschritt wird der ungesättigte Aldehyd zum entsprechenden gesättigten Alkohol, dem 3-(4-Chlorphenyl)-2-methylpropanol hydriert. Die Umsetzung mit Wasserstoff erfolgt in der Flüssigphase in Gegenwart eines Nickel enthaltenden Katalysators. Es hat sich bewährt, solche Katalysatoren einzusetzen, die 20 bis 60 und insbesondere 40 bis 55 Gew.-% Nickel (bezogen auf den Gesamtkatalysator), daneben Träger und gegebenenfalls Aktivatoren enthalten. Als Trägermaterial ist Aluminiumoxid oder Kie selgur geeignet. Bevorzugt wird ein 50 bis 55 Gew.-% Nickel und daneben Kieselgur als Träger enthaltender Katalysator. In Abhängigkeit von der Zusammensetzung des Katalysators liegt die Hydriertemperatur im allgemeinen im Bereich von 80 bis 120°C. Bei 95 bis 97 %igem Umsatz des eingesetzten ungesättigten Aldehyds erhält man 3-(4-Chlorphenyl)-2-methylpropanol mit einer Selektivität von 90 % und mehr.

Nach Abtrennung des Hydrierkatalysators kann der Alkohol zwar unmittelbar, d.h. wiederum ohne vorherige Reinigung der Dehydratisierungsreaktion unterworfen werden. In manchen Fällen ist es jedoch zweckmäßig, den Alkohol zuvor durch eine grobe Destillation vorzureinigen, so daß er in einer Reinheit >98 % vorliegt. Die Dehydratisierung des 3-(4-Chlorphenyl)-2-methylpropanols zu 1-(4-Chlorphenyl)-2-methylpropen-1 und -propen-2 erfolgt in der Gasphase bei Temperaturen zwischen 280 und 340°C in Gegenwart eines vorwiegend Aluminiumoxid enthaltenden Katalysators. Mit besonderem Erfolg werden Katalysatoren eingesetzt, die außer Aluminiumoxid z.B. noch Siliciumdioxid enthalten. Überraschenderweise verläuft die Dehydratisierung des Alkohols in Gegenwart solcher Katalysatoren, ohne daß Produkte mit isomerisiertem Kohlenstoffgerüst entstehen, die bei der Verwendung anderer Katalysatoren in erheblichem Umfange auftreten. Bei Temperaturen von 310 bis 330°C und einer Raumgeschwindigkeit von 0,1 bis 0,5 m³/h . m³ erzielt man einen Umsatz von >95 %. Das Produkt wird zur Erhöhung des I-(4-Chlorphenyl)-2-methylpropen-1-Anteils in Gegenwart eines sauren Katalysators, vorzugsweise Schwefelsäure, isomerisiert und darauf destillativ an einer Kolonne mit 20 bis 60 theoretischen Böden aufgearbeitet. 1-(4-Chlorphenyl)-2-methylpropen-1 fällt, nach Destillation, in einer Ausbeute von mehr als 65 %, bezogen auf den eingesetzten Alkohol, an.

Das durch Dehydratisierung des Alkohols erhaltene Propylenderivat kann zwar ohne vorherige Aufarbeitung oder Reinigung unmittelbar der Hydroformylierung zugeführt werden. Vorzugsweise sollte jedoch ein durch fraktionierte Destillation gereinigtes Olefin in die Hydroformylierungsreaktion eingesetzt werden. Die Reaktion wird, wie üblich, in Gegenwart von Carbonyle bildenden Metallen der 8. Gruppe des Periodensystems durchgeführt. Besonders bewährt hat sich die Verwendung von Rhodium als Katalysator, das in Form eines Rhodiumcarbonylkomplexes im Reaktionsgemisch enthalten ist. In der Praxis werden in einem Druckgefäß das substituierte Propylen, ein Rhodium-Salz, z.B. Rhodiumchlorid oder Rhodium-2-ethylhexanoat vorgelegt. Die Rh-Konzentration, bezogen auf das eingesetzte Olefin, beträgt 10 bis 1000 ppm. Kohlenmonoxid und Wasserstoff werden zweckmäßig im Verhältnis 1 : 1 dem Reaktor zugeführt, die Umsetzung läuft bei 100 bis 160°C und einem Druck von 15 bis 30 MPa ab. Das Kohlenmonoxid/Wasserstoff-Gemisch wird dem Reaktor in dem Maße zugeführt, daß der gewählte Reaktionsdruck aufrechterhalten bleibt. Die Verwendung von Rhodiumkatalysatoren führt zu dem Ergebnis, daß das Olefin nahezu vollständig zu einem Gemisch aus 2-(4-Chlorphenyl)-3-methylbutanal und 3-(4-Chlorphenyl)-2,2-dimethylpropanal umgesetzt wird. Alkohole entstehen nicht. Darüber hinaus werden keine weiteren Nebenprodukte durch Isomerisierung des Ausgangsolefins gebildet.

Das Hydroformylierungsprodukt wird in bekannter Weise durch Abtrennung des Katalysators und Destillation aufgearbeitet. Es resultiert ein Gemisch aus 96 bis 97 Gew.-% 2-(4-Chlorphenyl)-3-methylbutanal und 3 bis 4 Gew.-% 3-(4-Chlorphenyl)-2,2-dimethylpropanal, das Verunreinigungen nur in untergeordnetem Maße enthält.

Dieses Aldehydgemisch wird mit Oxidationsmitteln wie Kaliumpermanganat, Wasserstoffperoxid, Natriumhypochlorit und anderen zu einem Gemisch der entsprechenden Säuren umgesetzt.

Die Umsetzungen erfolgen z.B. bei Verwendung von Kaliumpermanganat als Oxidationsmittel und in Gegenwart von verdünnter Schwefelsäure bei Temperaturen von 10 bis 30°C. Das Oxidationsmittel wird in einer Menge von 1 bis 1,5 mol pro mol Aldehyd eingesetzt. Ähnlich verfährt man, wenn die Oxidation mit Natriumhypochlorit oder Wasserstoffperoxid erfolgt.

Das Reaktionsgemisch wird nach Beendigung der Umsetzung mit einem geeigneten Lösungsmittel, bevorzugt werden aromatische Kohlenwasserstoffe wie Toluol oder Xylol, versetzt. Man trennt die organische Phase ab und befreit sie in einem Rotationsverdampfer im Vakuum vom Lösungsmittel. Zur Reinigung wird der Rückstand umkristallisiert. Bewährt hat sich hierfür z.B. Cyclohexan.

Das erfindungsgemäße Verfahren wird in den nachfolgenden Beispielen näher beschrieben.

Beispiel 1:

Herstellung von 3-(4-Chlorphenyl)-2-methylpropenal

In einem mit Rührer, Rückflußkühler, Innenthermometer und Tropftrichter ausgerüsteten 2 1-Dreihalskolben werden 500 g (3,56 mol) 4-Chlorbenzaldehyd und 569,6 g (0,712 mol) 5 %ige Natronlauge unter N₂-Schutz vorgelegt und auf 100°C erwärmt. Innerhalb von einer Stunde tropft man bei etwa 100°C 227,8 g (3,93 mol) Propionaldehyd zu und läßt unter Rückfluß bei etwa 103°C eine Stunde nachreagieren. Darauf gibt man 356 g Toluol zu, kühlt den Kolbeninhalt auf etwa 50°C und trennt die Phasen. Man erhält eine organische Phase (1031,3 g) und eine Wasserphase (622,1 g). Die organische Phase wird einmal mit 178 g Wasser gewaschen. Nach erneuter Phasentrennung erhält man eine Waschwasserphase (199,4 g) und die Wertproduktphase (1009,9 g), die gaschromatographisch analysiert wird.

```
GLC-Analyse:
    Toluol                                   41,05 Gew.-%
    2-Methylpentenal                          1,84 Gew.-%
    4-Chlorbenzaldehyd                        3,16 Gew.-%
    3-(4-Chlorphenyl)-2-methylpropenal       52,68 Gew.-%
    Sonstige                                  1,27 Gew.-%
```

Hieraus ergibt sich die 3-(4-Chlorphenyl)-2-methylpropenal-Ausbeute zu 90,8 % der Theorie, bezogen auf 4-Chlorbenzaldehyd.

Beispiele 2 bis 8:

Weitere Untersuchungen zur Herstellung von 3-(4-Chlorphenyl)-2-methylpropanal

Analog Beispiel 1 werden unter den in Tabelle 1 angegebenen Bedingungen weitere Umsetzungen zur Herstellung von 3-(4-Chlorphenyl)-2-methylpropenal durchgeführt.

EP 0 436 860 A1

Tabelle 1:

| Bei-spiel | Einsatzverhältnis (mol) 4-Chlor-benz-aldehyd | Propion-aldehyd | NaOH | NaOH-Konz. (%) | Temp. (°C) | Lösungs-mittel | GLC-Analyse (in Gew.-%) 1 | 2 | 3 | 4 | 5 | Aus-beute (% der Theor.) |
|---|---|---|---|---|---|---|---|---|---|---|---|---|
| 2 | 1 | 1,15 | 0,20 | 5,0 | 95 | Toluol | 40,44 | 2,61 | 2,86 | 50,77 | 3,32 | 87,7 |
| 3 | 1 | 1,20 | 0,20 | 5,0 | 100 | Toluol | 40,87 | 3,26 | 2,11 | 51,12 | 2,64 | 88,7 |
| 4 | 1 | 1,25 | 0,20 | 5,0 | 100 | Toluol | 40,13 | 3,85 | 2,08 | 51,41 | 2,53 | 94,8 |
| 5 | 1 | 1,20 | 0,25 | 5,0 | 100 | Toluol | 40,79 | 3,18 | 1,73 | 51,63 | 2,67 | 88,8 |
| 6 | 1 | 1,20 | 0,20 | 7,5 | 103 | Toluol | 41,16 | 3,26 | 2,03 | 51,00 | 2,55 | 93,1 |
| 7 | 1 | 1,20 | 0,15 | 7,5 | 103 | Toluol | 41,12 | 2,85 | 1,70 | 51,84 | 2,49 | 90,4 |
| 8 | 1 | 1,20 | 0,20 | 5,0 | 102 | Cyclo-hexan | 39,87 | 2,70 | 1,40 | 54,34 | 1,69 | 92,1 |

1: Lösungsmittel

2: 2-Methylpentenal

3: 4-Chlorbenzaldehyd

4: 3-(4-Chlorphenyl)-2-methylpropenal

5: Sonstige

Beispiel 9:

Herstellung von 3-(4-Chlorphenyl)-2-methylpropanol

Zur Hydrierung von 3-(4-Chlorphenyl)-2-methylpropenal wird ein Aldolisierungsprodukt folgender Zusammensetzung verwendet:

```
Toluol                                    41,08 Gew.-%

2-Methylpentenal                           2,56 Gew.-%

4-Chlorbenzaldehyd                         3,32 Gew.-%

3-(4-Chlorphenyl)-2-methylpropenal        51,01 Gew.-%

Sonstige                                   2,03 Gew.-%
```

In einem 1 1-Autoklav werden 500 g Aldolisierungsprodukt obiger Zusammensetzung, 25 g Nickelkatalysator (52 Gew.-% Ni auf Träger) bei 90°C und 10 MPa Wasserstoffdruck hydriert, die Reaktionszeit beträgt etwa 6 Stunden.

Nach Abkühlen des Reaktorinhaltes wird der Katalysator abgetrennt und die organische Phase (502 g) gaschromatographisch analysiert.

```
GLC-Analyse:
    Toluol                                47,82 Gew.-%

    2-Methylpentanol                       1,26 Gew.-%

    4-Chlorbenzylalkohol                   1,85 Gew.-%

    3-(4-Chlorphenyl)-2-methylpropanol    44,07 Gew.-%

    Sonstige                               5,00 Gew.-%
```

Die gaschromatographisch bestimmte Ausbeute beträgt 86,8 % der Theorie.

Beispiel 10:

Herstellung von 1-(4-Chlorphenyl)-2-methylpropen-1

Die Dehydratisierung des Hydrierproduktes (aus Beispiel 9) erfolgt an 300 ml eines gamma-$Al_2O_3$-Katalysators, der in einem 120 cm langen Reaktionsrohr angeordnet ist. Über der Katalysatorzone (etwa 80 cm) ist eine 30 cm hohe Schüttung aus Raschigringen als Verdampferzone angeordnet. Nach Erreichen der Reaktionstemperatur von 320°C in dem gleichmäßig beheizten Ofen werden mittels einer Dosierpumpe 5000 g des Hydrierproduktes aus Beispiel 9 mit einer Geschwindigkeit von 0,2 V/Vh über Kopf in das Rohr eingebracht. Dabei verdampft das Produkt in der Raschigring-Zone und trifft gasförmig auf den Katalysator.

Am unteren Ausgang des Reaktionsrohres befindet sich ein Intensivkühler, in dem das Reaktionsgemisch kondensiert und in den abschließenden Rundkolben tropft.

Nach Phasentrennung wird das organische Produkt gaschromatographisch analysiert.

<u>GLC-Analyse:</u>

| | |
|---|---|
| Toluol | 44,49 Gew.-% |
| 1-(4-Chlorphenyl)-2-methylpropen-2 | 32,86 Gew.-% |
| 1-(4-Chlorphenyl)-2-methylpropen-1 | 12,42 Gew.-% |
| 3-(4-Chlorphenyl)-2-methylpropanol | 2,30 Gew.-% |
| Sonstige | 7,92 Gew.-% |

Nach Destillation an einer Kolonne mit 24 theoretischen Böden erhält man bei einem Rücklaufverhältnis von 3 : 1 und einer Kopftemperatur von 103 bis 113°C/3 kPa eine Hauptfraktion folgender Zusammensetzung:

| | |
|---|---|
| 1-(4-Chlorphenyl)-2-methylpropen-2 | 71,92 Gew.-% |
| 1-(4-Chlorphenyl)-2-methylpropen-1 | 25,69 Gew.-% |
| Sonstige | 2,39 Gew.-% |

In einem 4 1-Rundkolben werden 1000 g des obigen Olefingemisches unter Zusatz von 1000 g 50 Gew.-%iger Schwefelsäure 9 Stunden bei 120°C isomerisiert; nach gaschromatographischer Analyse enthält das Reaktionsprodukt danach noch 18,31 Gew.-% 1-(4-Chlorphenyl)-2-methylpropen-2 und 76,94 Gew.-% 1-(4-Chlorphenyl)-2-methylpropen-1 sowie 4,75 Gew.-% sonstige Nebenprodukte.

Die destillative Aufarbeitung an einer Kolonne mit 24 theoretischen Böden führt zu 1392 g einer Hauptfraktion, die 94,33 Gew.-% 1-(4-Chlorphenyl)-2-methylpropen-1 und daneben noch 2,51 Gew.-% 1-(4-Chlorphenyl)-2-methylpropen-2 und 3,16 Gew.-% sonstige Nebenkomponenten enthält.

Die Ausbeute beträgt 66,0 % der Theorie, bezogen auf eingesetztes 3-(4-Chlorphenyl)-2-methylpropanol.

Beispiel 11:

Herstellung von 2-(4-Chlorphenyl)-3-methylbutanal

Ein nach Beispiel 10 hergestelltes 1-(4-Chlorphenyl)-2-methylpropen-1 wird im Gewichtsverhältnis 1 : 1 mit Cyclohexan gemischt; 1100 g der Mischung werden in einem mit einem Hubrührer ausgestatteten 2 1 Autoklav bei 120°C, 27 MPa Druck in Gegenwart von 100 ppm Rhodium (bezogen auf das substituierte Propen-1) als Katalysator, hydroformyliert. Nach etwa 14 Stunden Reaktion wird der Autoklaveninhalt (1187 g) abgekühlt und gaschromatographisch analysiert.

<u>GLC-Analyse:</u>

| | |
|---|---|
| Cyclohexan | 52,67 Gew.-% |
| 1-(4-Chlorphenyl)-2-methylpropen-1 | 2,05 Gew.-% |
| 2-(4-Chlorphenyl)-3-methylbutanal | 32,42 Gew.-% |
| 3-(4-Chlorphenyl)-2,2-dimethylpropanal | 9,15 Gew.-% |
| Sonstige | 3,71 Gew.-% |

Das Hydroformylierungsprodukt wird an einem Dünnschichtverdampfer zweistufig destilliert. Bei einer Manteltemperatur von 140°C und unter Normaldruck trennt man zunächst das Cyclohexan ab und destilliert den verbleibenden Rückstand (925,7 g $\hat{=}$ 78,0 % vom Einsatz) danach bei einer Manteltemperatur von 180°C und 2 kPa.

Das Destillat (648,0 g $\hat{=}$ 54,6 % vom Einsatz) enthält 64,4 Gew.-% 2-(4-Chlorphenyl)-3-methylbutanal, 19,20 Gew.-% 3-(4-Chlorphenyl)-2,2-dimethylpropanal, 4,45 Gew.-% Cyclohexan und 11,95 Gew.-% sonstige Nebenkomponenten.

Die folgende fraktionierte Destillation an einer Kolonne mit 24 theoretischen Böden, Rücklaufverhältnis 3 : 1, führt zu einer bei 115 bis 117° C, 7 mbar) siedenden Hauptfraktion (401,7 g $\hat{=}$ 33,8 % vom Einsatz) mit einem Gehalt von 96,42 Gew.-% 2-(4-Chlorphenyl)-3-methylbutanal.

Die Ausbeute beträgt demnach 69,6 % der Theorie, bezogen auf eingesetztes 1-(4-Chlorphenyl)-2-methylpropen-1.

Beispiel 12:

Herstellung von 2-(4-Chlorphenyl)-3-methylbuttersäure

In einem mit Rührer, Rückflußkühler, Tropftrichter und Innenthermometer ausgerüsteten 1 1-Dreihalskolben werden 100 g (0,49 mol) 96,42 Gew.-%iges 2-(4-Chlorphenyl)-3-methylbutanal und 558,4 g 19,4 Gew.-%ige Schwefelsäure vorgelegt und bei 20 bis 25° C innerhalb von 2 Stunden mit 79,05 g (0`5 mol) KMnO$_4$ versetzt. Nach einer Stunde Nachreaktion bei 20 bis 25° C werden 600 g Toluol zugegeben. Der Feststoff wird abfiltriert, die organische Phase von der Wasserphase abgetrennt. Die organische Phase wird am Rotationsverdampfer vom Toluol befreit, der Rückstand aus Cyclohexan umkristallisiert.

Die Ausbeute beträgt 86,4 g $\hat{=}$ 82,8 % der Theorie (bezogen auf eingesetzten Aldehyd), das Produkt hat einen Schmelzpunkt von 89 bis 91° C.

**Patentansprüche**

1. Verfahren zur Herstellung von 2-(4-Chlorphenyl)-3-methylbuttersäure, dadurch gekennzeichnet, daß man 4-Chlorbenzaldehyd mit Propionaldehyd zu 3-(4-Chlorphenyl)-2-methylpropenal umsetzt, das Propenalderivat unter Bildung von 3-(4-Chlorphenyl)-2-methylpropanol hydriert, den Alkohol dehydratisiert, wobei 1-(4-Chlorphenyl)-2-methylpropen-1 entsteht, dieses substituierte Propylen hydroformyliert und den dabei entstehenden 2-(4-Chlorphenyl)-2-methylbutyraldehyd zur 2-(4-Chlorphenyl)-3-methylbuttersäure oxidiert.

2. Verfahren nach Anspruch 1, dadurch gekennzeichnet, daß bei der Aldolkondensation 4-Chlorbenzaldehyd mit Propionaldehyd im Molverhältnis 1 : 1 bis 1 : 2, vorzugsweise 1 : 1,1 bis 1 : 1,3 eingesetzt wird und als Katalysator Natriumhydroxid in einer Menge von 0,1 bis 0,5 mol je mol 4-Chlorbenzaldehyd verwendet wird.

3. Verfahren nach Anspruch 1 oder 2, dadurch gekennzeichnet, daß das Aldolisierungsprodukt 3-(4-Chlorphenyl)-2-methylpropanol in Gegenwart eines 20 bis 60, insbesondere 40 bis 55 Gew.-% Nickel (bezogen auf den Gesamtkatalysator) enthaltenden Katalysators in der Flüssigphase hydriert wird.

4. Verfahren nach Anspruch 3, dadurch gekennzeichnet, daß der Katalysator 50 bis 55 Gew.-% Nickel und daneben Kieselgur als Träger enthält.

5. Verfahren nach einem oder mehreren der Ansprüche 1 bis 4, dadurch gekennzeichnet, daß das Hydrierprodukt 3-(4-Chlorphenyl)-2-methylpropanol in Gegenwart eines vorwiegend Aluminiumoxid enthaltenden Katalysators bei 280 bis 340° C zu einem Gemisch aus 1-(4-Chlorphenyl)-2-methylpropen-1 und 1-(4-Chlorphenyl)-2-methylpropen-2 dehydratisiert wird.

6. Verfahren nach Anspruch 5, dadurch gekennzeichnet, daß die Dehydratisierung bei 310 bis 330° C erfolgt und der Alkohol mit einer Raumgeschwindigkeit von 0,1 bis 0,5 m³/h . m³ über den Katalysator geleitet wird.

7. Verfahren nach Anspruch 5 oder 6, dadurch gekennzeichnet, daß das Dehydratisierungsgemisch in Gegenwart eines sauren Katalysators, vorzugsweise Schwefelsäure, isomerisiert wird.

8. Verfahren nach einem oder mehreren der Ansprüche 1 bis 7, dadurch gekennzeichnet, daß das Dehydratisierungsprodukt 1-(4-Chlorphenyl)-2-methylpropen-1 bei 100 bis 160° C und 15 bis 30 MPa Druck in Gegenwart von 10 bis 1000 ppm Rhodium (bezogen auf das Olefin) hydroformyliert wird.

9. Verfahren nach einem oder mehreren der Ansprüche 1 bis 8, dadurch gekennzeichnet, daß das Hydroformylierungsprodukt bei 10 bis 30° C mit Kaliumpermanganat, Wasserstoffperoxid oder Natriumhypochlorit zu der 2-(4-Chlorphenyl)-3-methylbuttersäure oxidiert wird.

| | EINSCHLÄGIGE DOKUMENTE | | EP 90123914.5 |
|---|---|---|---|
| Kategorie | Kennzeichnung des Dokuments mit Angabe, soweit erforderlich der maßgeblichen Teile | Betrifft Anspruch | KLASSIFIKATION DER ANMELDUNG (Int Cl⁵) |
| A | CHEMICAL ABSTRACTS, Band 105, Nr. 9, 1. September 1986, Columbus, Ohio, USA B. PURSHOTAM et al. "4-Chloro--alpha-(1-methylethyl)benzene-acetic acid from 4-chloro--alpha-(1-methylethyl)benzene-acetaldehyde using hypohalites of alkali metals or alkaline earth metals" Seite 620, Spalte 1, Zusammen-fassung-Nr. 78 666c & Indian IN 155,767 -- | 1,9 | C 07 C 57/58 C 07 C 51/00 |
| D,A | CHEMICAL ABSTRACTS, Band 105, Nr. 9, 1. September 1986, Columbus, Ohio, USA B. PURSHOTAM et al. "4-Chloro--(1-methylethyl)benzeneacetic acid from 1-(4-chlorophenyl)--2-methylpropan-1-one" Seite 619, Spalte 2, Zusammen-fassung-Nr. 78 660w & Indian IN 155,639 -- | 1,9 | |
| | | | RECHERCHIERTE SACHGEBIETE (Int Cl⁵) |
| A | CHEMICAL ABSTRACTS, Band 82, Nr. 25, 23. Juni 1975, Columbus, Ohio, USA K. FUJIMOTO et al. "Substi-tuted phenylacetic acids" Seite 489, Spalte 2, Zusammen-fassung-Nr. 170 399x & Japan. Kokai 75 05,350 ---- | 1 | C 07 C 51/00 C 07 C 57/00 |

Der vorliegende Recherchenbericht wurde für alle Patentansprüche erstellt

| Recherchenort | Abschlußdatum der Recherche | Prüfer |
|---|---|---|
| WIEN | 21-03-1991 | HOFBAUER |